# EUROPEAN PATENT APPLICATION

(11) **EP 0 529 775 A1**
(43) Date of publication of application: **03.03.1993**
(21) Application number: 92305913.3
(22) Date of filing: 26.06.1992
(51) Int. Cl.: G01N 33/543, G01N 33/553

(54) **Arraying process of ultra-small objects and fabrication process of ultra-fine structure by using the arraying process**

(30) Priority: 28.06.1991 JP 158134/91; 30.08.1991 JP 219078/91; 18.12.1991 JP 334626/91; 18.03.1992 JP 62130/92
(71) Applicant: Oki Electric Industry Co., Ltd., Tokyo (JP)
(72) Inventor: Koyano, Takeshi, Minato-ku, Tokyo (JP); Saito, Minoru, Minato-ku, Tokyo (JP); Miyamoto, Yasuo, Minato-ku, Tokyo (JP); Kaifu, Katsuaki, Minato-ku, Tokyo (JP); Kato, Masakazu, Minato-ku, Tokyo (JP)
(74) Representative: Read, Matthew Charles

(57) **Abstract**

A substrate is immersed in an aqueous solution of aminopropyltriethoxysilane to bind amino groups on the substrate. Certain amino groups are selectively eliminated by an electron beam so that an array pattern of amino groups is obtained. The substrate with the array pattern formed thereon is then immersed in a bicarbonate buffer in which biotin is contained, whereby biotin molecules are coupled to the amino groups in the form of the array pattern. The resulting substrate is immersed in an avidin-containing bicarbonate buffer. On the side, biotin is added to a suspension of liposome with alamethicin incorporated therein, whereby a biotin molecule is coupled to the amino group of each alamethicin molecule to obtain a suspension of biotinylated proteoliposome. In this suspension, the avidin-bound substrate is immersed. Biotinylated proteoliposome particles are immobilized firmly at a high density on the substrate via the respective avidine molecules.

## Description

### BACKGROUND OF THE INVENTION

### 1) Field of the Invention

This invention relates to a process for forming an array pattern of ultrasmall objects on a base and also to a process for fabricating an ultrafine structure by using the forming process.

### 2) Description of the Related Art

In various fields and for various purposes, it is practiced to immobilize or otherwise fix ultrasmall objects in a desired array pattern on a base.

For example, co-assigned Japanese Patent Application Laid-Open (Kokai) No. SHO 63-111428 discloses a process for two-dimensionally arraying liposome particles, which have a certain function as ultrasmall objects, on a substrate as a base in order to obtain a biochip simulating a function of an organism. Described specifically, antibody molecules are immobilized on a substrate by the Langmuir-Blodgett (LB) method. An electron beam or ion beam is selectively illuminated onto the immobilized antibody molecules, whereby the antibody molecules located at unnecessary regions are selectively eliminated to form a desired pattern of the antibody molecules on the substrate. The liposome particles referred to above are then immobilized on the antibody pattern by using the antigen-antibody reaction.

Further, Japanese Patent Application Laid-Open (Kokai) No. HEI 3-79774 discloses a process for arraying, as ultrasmall objects, ultrafine particles of a metal, metal compound, ceramic, oxide superconductor or organic substance in order on a substrate as a base so that various electronic devices can be obtained. Described specifically, molecules of an antigen are two-dimensionally arrayed in order on a substrate. On the side, molecules of a monoclonal antibody which reacts to the antigen are bound with ultrafine particles. The ultrafine particles with the antibody molecules bound thereon are then bound to the substrate on which the antigen molecules have been arrayed.

Each of the above prior art processes, however, makes use of the antigen-antibody reaction for the arrayed immobilization of the ultrasmall objects on the base, so that the binding strength or affinity between the ultrasmall objects and the base is not fully satisfactory. In addition, a limitation is naturally imposed whenever one wants to immobilize ultrasmall objects at a high density on a base.

On the other hand, research is under way in recent years with a view toward constructing a new device by using a protein, because the provision of such as device involves the possibility that the excellent information processing ability of the cranial nerve system of an organism may be realized artificially. This, however, requires arrayed immobilization of molecules of a function protein on a substrate. No process has however yet been developed to permit such immobilization, leading to the outstanding desire for the development of a process therefor.

In electronic industry, there is an ever-increasing demand for the development of a technique which enables to form still finer conductive paths. With a view toward meeting this demand, it is practiced to conduct the formation of conductive paths by a new technique such as electron beam lithography or X-ray lithography in place or conventional, wide-used optical lithography. Even with these lithographic technology, it is not possible to form conductive paths finer than the quarter micron rule under the circumstances. There is, accordingly, an outstanding desire for the development of a process which easily permits formation of conductive paths with a line width of several tens nanometers or smaller.

The 1990 Catalog of MELLES GRIOT COMPANY, for example, discloses a process for forming a polarizer by arraying a number of narrow lines, which are formed of thin films of metal whiskers, side by side at a prescribed internal on a glass substrate as a base in accordance with a lithographic technique. This polarizer performs polarization of light in a similar mechanism to conventionally-known, so-called wire grid polarizers fabricated by arranging a number of wires side by side, so that the internal of the narrow lines formed of the thin films of the metal whiskers is required to have a dimension suited for the wavelength of light to be polarized. There is, however, a limitation to the reduction of the internal of the narrow lines whichever presently-available technique is employed. As is shown in the above catalog, polarizers for near infrared light - such as polarizers for light of 750-1250 nm wavelength and polarizers for light of 1250-1550 nm wavelength - are only available these days. Since fabrication of a polarizer for the ultraviolet range of still shorter wavelengths requires to reduce the interval of the narrow lines to 200-400 nm or so, it is desired to develop a technique which permits a further reduction in the internal of narrow metal lines.

### OBJECTS OF THE INVENTION

With the foregoing in view, the present invention has now been completed.

A first object of this invention is, therefore, to provide a process for firmly immobilizing ultrasmall objects at a high density in a predetermined array pattern on a base.

A second object of this invention is to provide a process for fabricating a biochip by firmly immobilizing liposome particles at a high density in a desired array pattern on a base.

A third object of this invention is to provide a process for arraying and firmly immobilizing ultrafine particles at a high density in a desired pattern on a base.

A fourth object of this invention is to provide a process for forming ultrafine conductive paths of a line width of several tens nanometers or less on a base.

A fifth object of this invention is to provide a process for fabricating a polarizer for short wavelengths, which polarizer has narrow metal lines arrayed side by side at a very small internal.

To attain the above objects, the present inventors have proceeded with a variety of investigation. As a result, it has come to mind that the specific binding reaction between avidin and biotin or the specific binding reaction between streptavidin and biotin, which has heretofore been used primarily as an immunohistochemical staining system for the identification of an antigen, could be used for the immobilization of ultrasmall objects on a base, because the binding force resulting from such a specific binding reaction is at least 1,000,000 times as strong as the binding force available through an antigen-antibody reaction. For further details of an immunohistochemical staining system making use of the specific binding reaction between avidin and biotin and of the binding force available by the reaction, reference may be had, for example, to the pamphlet entitled "HISTOPHINE SABPO KIT" and distributed by NICHIREI CORPORATION (prepared in December, 1989).

### SUMMARY OF THE INVENTION

The present invention therefore provides, in one aspect thereof, a process for arraying ultrasmall objects, which comprises immobilizing the ultrasmall objects in a predetermined pattern on a base by using at least one of a specific binding reaction between avidin and biotin and a specific binding reaction between streptavidin and biotin.

In the first aspect of this invention, the term "base" can embrace a variety of solid bases usable for arraying ultrasmall objects. Examples of the base include semiconductor substrates employed for the fabrication of semiconductor devices, such as silicon substrate and compound semiconductor substrates; glass substrates useful for the fabrication of polarizers; and various substrates made of an inorganic or organic substance and useful for the fabrication of electronic elements or devices. These substrates may also be those carrying one or more other elements already formed thereon in advance.

In a preferred embodiment of the first aspect of this invention, a desired array pattern of first biotin molecules is first formed on a base. Second biotin molecules are bound to ultrasmall objects in advance. The ultrasmall objects with the second biotin molecules bound thereon are then coupled via a streptavidin or avidin molecule to the first biotin molecules arrayed in the desired pattern on the base. In another preferred embodiment, a desired array pattern of biotin molecules is first formed on a base. Streptavidin or avidin molecules are bound to ultrasmall objects in advance. The ultrasmall objects with the streptavidin or avidin molecules bound thereon are then coupled to the biotin molecules arrayed in the desired pattern on the base.

In the first aspect of this invention, the formation of the array pattern of biotin molecules on the base can be conducted preferably by binding amino groups to the entire surface of the base, patterning the thus-bound amino groups and then coupling biotin molecules to the resulting patterned amino groups or by binding amino groups to the entire surface of the base, coupling biotin molecules to the thus-bound amino groups and then patterning the thus-coupled biotin molecules. The patterning of these amino groups or biotin molecules can be carried out by selectively illuminating, for example, an electron beam, a laser beam, ultraviolet rays or X-rays onto the amino groups or biotin molecules formed on the entire surface of the base.

The process according to the second aspect of this invention is to fabricate a biochip. It features conducting each processing of the first aspect by using protein molecules as the ultrasmall objects in the first aspect. Preferably, each processing of the first aspect is conducted using as the protein molecules liposome particles formed of at least one of biosubstances and biosubstance analogues. Upon practice of the second aspect, it is preferable to incorporate in advance at least one membrane protein molecule as a biotin binding site in the lipid bilayer of each of the above-mentioned liposome particles and then to couple a liposome-side biotin molecule to the membrane protein molecule.

The process according to the third aspect of this invention is to array ultrafine particles. It features conducting each processing of the first aspect by using ultrafine particles as the ultrasmall objects in the first aspect. Various kinds of ultrafine particles can be used as the ultrafine particles depending on the purpose. Illustrative ultrafine particles include ultrafine particles of metals such as Au, Ag, Ni, Co, Fe, Cu and Pd; ultrafine particles of metal compounds such as WCl₆, W₂C, Fe₃O₄, γ-Fe₂O₃ and CrO₂; ultrafine particles of ceramics such as BaTiO₃ and MnFe₂O₄; ultrafine particles of high-temperature oxide superconductors such as YBa₂Ca₃O₇; and ultrafine particles of organic substances such as carbon.

The process according to the fourth aspect of this invention is to form ultrafine conductive paths. It features conducting each processing of the first aspect by using colloidal metal particles as the ultrasmall objects in the first aspect.

The process according to the fifth aspect of this invention is to fabricate a polarizer. It features conducting each processing of the first aspect by using colloidal metal particles as the ultrasmall objects in the first aspect.

According to the features of the first to fifth aspects of the present invention, the immobilization of ultrasmall objects on a base is effected using the specific binding reaction between avidin and biotin and/or the specific binding reaction between streptavidin and biotin so that the strength of immobilization of the ultrasmall objects on the base has been improved over the strength of immobilization of ultrasmall objects on a base by an antigen-antibody reaction.

The features of these aspects of the present invention have also made it possible to immobilize ultrasmall objects on a base at a higher density that their immobilization by an antigen-antibody reaction as is apparent from experiment results to be described subsequently, although reasons for this difference are not certain.

Where biotin molecules are bound to a base via an amino group, a covalent bond is formed between each biotin molecule and its corresponding amino group. Since OH groups are inevitably present on the surface of the base in many instances and, even if not present, it is easy to apply OH groups to the surface of the base, the base and the amino groups are also bound covalently. As a result, each biotin molecule is bound with the base via covalent bonds. Where each biotin molecule is bound with the base via covalent bonds, their binding is far stronger than that available from the conventional technique that an antibody is immobilized on a base by the LB method. Sufficient strength is therefore assured for the binding other than the binding between each avidin (streptavidin) molecule and its corresponding biotin molecule, whereby the specific binding between avidin and biotin and/or the specific binding between streptavidin and biotin can be used more effectively.

Where an electron beam or the like is selectively illuminated to amino groups or biotin molecules, binding activity remains at the biotin molecules or amino groups not exposed to the electron beam. The biotin molecules or amino groups which still retain binding activity hence function as a biotin molecule pattern. As has been described above, biotin molecules can themselves be patterned like a resist employed extensively in lithography. Moreover, the resolution of a pattern of biotin molecules so arrayed can be directly controlled, for example, by the diameter of an electron beam so that the resolution can be made much finer than that of a conventional resist pattern. Because ultrasmall objects are arrayed in accordance such a fine pattern of biotin molecules, it is possible to form an array pattern of several tens nanometers in line width although such a fine array pattern is unavailable by conventional lithography.

For the less non-specificity of the specific binding reaction between streptavidin and biotin than the specific binding reaction between avidin and biotin, the use of the former reaction (the specific binding reaction between streptavidin and biotin) is considered to permit more selective binding of ultrasmall objects to a biotin molecule pattern formed on a base compared with the use of the latter reaction.

When ultrasmall objects are immobilized on a base by using an antigen-antibody reaction, the binding between the ultrasmall objects and the base is unstable (reversible) due to variations in the pH of a buffer solution. In contrast, the invention of the present application makes use of the above-mentioned specific binding reaction in each aspect so that the binding between a base and ultrafine objects can be made irreversible.

The process according to the second aspect of this invention is to fabricate a biochip. It features advance .incorporation of at least one membrane protein molecule as a binding site for a biotin molecule in the lipid bilayer of each liposome particle, followed by coupling of a liposome-side biotin molecule with the membrane protein molecule. The liposome particle is therefore covalently bound with the biotin molecule via an amino group contained in the membrane protein molecule and, moreover, the membrane protein molecule is firmly incorporated in structure in the liposome particle, so that sufficient binding strength is secured between the liposome particle and the biotin molecule. As a consequence, sufficient binding strength is secured for the bond other than that between each avidin (streptavidin) molecule and its corresponding biotin molecule, thereby making it possible to more effectively use the specific binding between avidin and biotin and/or the specific binding between streptavidin and biotin.

### BRIEF DESCRIPTION OF THE DRAWINGS

FIG. 1 is a schematic illustration of biotinylated proteoliposome, and will be employed in the description of one example of the process according to this invention for the fabrication of a biochip;
FIG. 2 is a schematic illustration of procedures for the formation of an array pattern of amino groups, and will be used in the description of the example of the process according to this invention for the fabrication of the biochip;
FIG. 3 shows a substrate with an array pattern of amino groups, and will be referred to upon description of another example of the process according to this invention for the fabrication of a biochip;
FIG. 4 illustrates a substrate with an array pattern of biotin molecules, and will be referred to upon description of a further example of the process according to this invention for the fabrication of a biochip;
FIG. 5 depicts a substrate with an array pattern of avidin molecules, and will be referred to upon description of a still further example of the process according to this invention for the fabrication of a biochip;
FIG. 6 is a schematic illustration of the biochip fabricated by the fabrication process in the example of FIG. 5;
FIG. 7 is a schematic illustration of biotinylated proteoliposome, and will be employed in the description of a still further example of the process according to this invention for the fabrication of a biochip;
FIG. 8 is a schematic illustration of procedures for the formation of an array pattern of biotin molecules, and will be used in the description of the still further example of the process according to this invention for the fabrication of the biochip;
FIG. 9 depicts the state of liposome particles arrayed and immobilized on a base by using the specific binding reaction between biotin and streptavidin, and will be referred to upon description of a still further example of the process according to this invention for the fabrication of a biochip;
FIG. 10 illustrates the state of protein molecules arrayed and immobilized on a base by using the specific binding reaction between biotin and avidin, and will be referred to upon description of a still further example of the process according to this invention for the fabrication of a biochip;
FIG. 11 will be referred to upon description of one example of the process of this invention for arraying ultrafine particles;
FIG. 12 schematically illustrates the state of metal paths formed using colloidal gold particles, and will be referred to upon description of one example of the process according to this invention for the formation of ultrafine conductive paths;
FIG. 13 schematically shows a method employed for the measurement of the I-V characteristic of metal paths formed by the example of the process according to this invention for the formation of ultrafine conductive paths;
FIG. 14 diagrammatically illustrates the results of the measurement of the I-V characteristic of the metal paths formed by the example of the process according to this invention for the formation of the ultrafine conductive paths;
FIG. 15 schematically depicts the state of immobilization of colloidal gold particles-streptavidin molecules on a substrate via a narrow line pattern of biotin molecules, and will be referred to upon description of one example of the process of this invention for the fabrication of a polarizer;
FIG. 16(A) is a plan view of the polarizer obtained in the example of FIG. 15 while FIG. 16(B) is a cross-section taken in the direction of lines XVI-XVI of FIG. 16(A), and FIGS. 16(A) and 16(B) will be referred to upon description of the example of the process of this invention for the fabrication of the polarizer;
FIG. 17 is a simplified schematic illustration of a system employed for the measurement of polarizing characteristics of the polarizer fabricated by example of the process of this invention for the fabrication of the polarizer;
FIG. 18 diagrammatically shows the angle of rotation vs. intensity of transmitted light characteristic of the polarizer fabricated by example of the process of this invention for the fabrication of the polarizer;
FIG. 19(A) schematically illustrate an open state formed by two polarizer while FIG. 19(B) schematically depicts a closed state formed by the two polarizer, and FIGS. 19(A) and 19(B) will be referred to upon description of the example of the process of this invention for the fabrication of the polarizer; and
FIG. 20 diagrammatically shows light transmission characteristics of the polarizers in the states of FIGS. 19(A) and 19(B), respectively, and will be referred to upon description of the example of the process of this invention for the fabrication of the polarizer.

### DETAILED DESCRIPTION OF THE INVENTION

The respective aspects of this invention will hereinafter be described with reference to the drawings. It is to be noted that the drawings to be referred to in the description of the invention merely illustrate the approximate dimensions and shapes of individual elements and their approximate positional relationships to such extents as enabling understanding of the respective aspects of this invention. In the drawings, like elements are identified by like reference numerals, and their description is omitted. It is also to be borne in mind that the reagents, the numerical conditions - such as the concentrations and amounts of the reagents, treatment times and treatment temperatures -, the treatment methods, and the like, all employed in the examples, are merely preferred examples within the scope of the present invention. It is, therefore, to be noted that the present invention is not limited to these conditions.

### A. Examples of the process for the fabrication of biochip:

First, examples of the process for the fabrication of a biochip useful as a taste sensor or smell sensor will be described.

### A-a. Where avidin and liposome were used:

A description will now be made of an example in which liposome particles are arrayed and immobilized on a base by using the specific binding reaction between avidin and biotin.

### A-a-1. Preparation of a suspension of biotinylated proteoliposome:

Liposome to be arrayed and immobilized on a base, said liposome being hereinafter to be called "proteoliposome" because liposome with a membrane protein incorporated therein is used in this example, is provided as will be described below. Employed as a lipid for the constitution of the liposome is dipalmitoylphosphatidylcholine (hereinafter abbreviated as "DPPC"; product of NOF CORPORATION) which is a biosubstance analogue. Further, alamethicin (product of Sigma Chemical Company) is used as the membrane protein to be incorporated in the lipid bilayer of the liposome. As the biotin to be coupled with the proteoliposome, sulfosucciimidyl 6-(biotinamido)hexanoate ("NHS-LC-BIOTIN", trade name; biotin derivative produced by Funakoshi, Ltd.) is used.

Placed and mixed first in an eggplant type flask of 50 mℓ capacity are 5 mℓ of a chloroform solution of DPPC, said chloroform solution containing DPPC at the concentration of 20 mg per mℓ of chloroform, and 1 mℓ of an ethanol solution of alamethicin, said ethanol solution containing alamethicin at the rate of 1 mg per mℓ of ethanol. The solvents are then driven off under reduced pressure from the liquid mixture by a rotary evaporator.

Next, 10 mℓ of bicarbonate buffer whose pH is 8.5 are added to the flask. The contents of the flask are then stirred at 37°C by a vortex mixer so that a suspension of proteoliposome is obtained. The existence of the proteoliposome in the suspension is confirmed by an optical microscope.

To avoid inclusion of proteoliposome particles of an unduly large particle size in the proteoliposome suspension, the proteoliposome suspension is next subjected to processing by "Extruder" (trade mark; liposome particle size classifier manufactured by NOF-Liposome Company). As a result, proteoliposome particles having a particle size not greater than 200 nm are obtained. This particle size classification is to facilitate determination of the quality of an array pattern of proteoliposome particles upon formation of the array pattern of the proteoliposome particles on a substrate.

To a suspension of the proteoliposome whose particle size has been classified, 1 mℓ of carbonate buffer (pH 8.5) containing 1 mg of "NHS-LC-BIOTIN" is next added. By stirring the proteoliposome suspension for 2 hours, the membrane protein (alamethicin in this case) of the proteoliposome is reacted with "NHS-LC-BIOTIN".

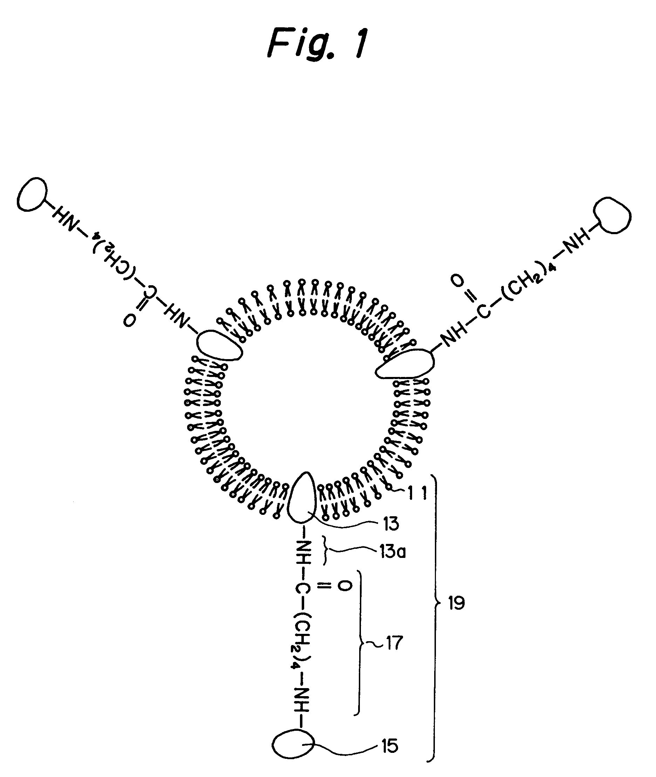

FIG. 1 schematically shows the structure of each proteoliposome particle after the reaction, in which there are depicted a lipid bilayer 11, membrane protein molecules (alamethicin molecules in this case) 13, amino groups 13a of the membrane protein molecules 13, biotin molecules 15, and spacer arms 17 derived from "NHS-LC-BIOTIN". This proteoliposome 19 will hereinafter be called "biotinylated proteoliposome" for the sake of convenience of description.

The biotinylated proteoliposome suspension is next diluted tenfold in bicarbonate buffer (pH 8.5). This is provided as an biotinylated proteoliposome suspension for use in experiments.

Any unduly high concentration of liposome in a biotinylated proteoliposome suspension involves the potential problem that biotinylated proteoliposome may be non-specifically bound to a substrate on which avidin molecules are to be arrayed in a pattern subsequently. The above dilution is to reduce this potential problem.

### A-a-2. Treatment of the substrate:

On the other hand, a glass substrate carrying biotin molecules arrayed in a pattern thereon is employed as a base on which biotinylated proteoliposome particles are to be immobilized. This glass substrate is provided in accordance with procedures as will be described below.

First, pre-treatment is applied to a glass substrate in the following manner.

A glass substrate of 1 cm x 1 cm wide is immersed for 2 minutes in an aqueous solution of aminopropyltriethoxysilane ("LS-3150", trade name; product of Shin-Etsu Silicone Co., Ltd.), which solution contained aminopropyltriethoxysilane at the concentration of 1% by volume. The glass substrate is pulled out of the aqueous solution and washed with purified water, and is then placed for 10 minutes in a constant-temperature chamber controlled at 110°C. By this series of treatments, amino groups to which "NHS-LC-BIOTIN" molecules are to be bound are immobilized on the surface of the glass substrate.

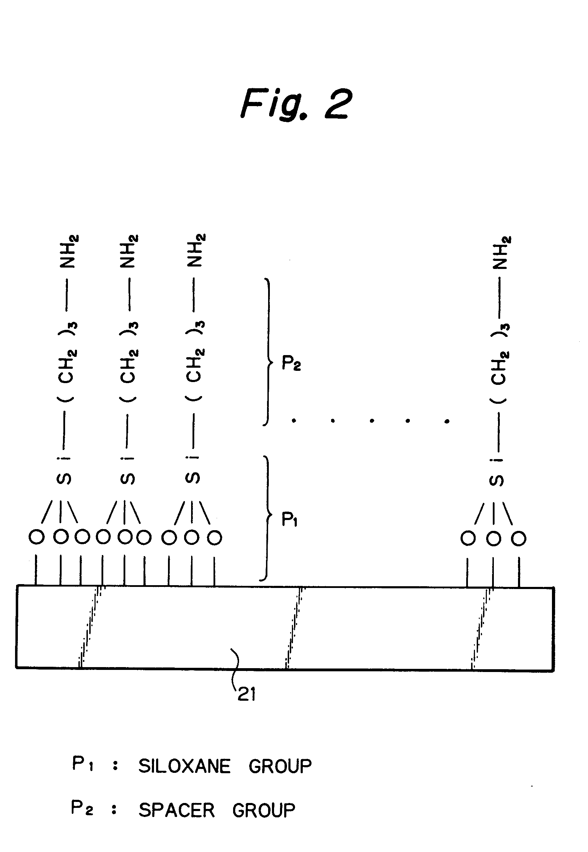

FIG. 2 schematically illustrates the glass plate 21 whose surface has been treated as described above. amino groups are immobilized on the entire surface of the glass substrate 21 via a siloxane group P₁ and a spacer arm P₂.

Onto the amino groups immobilized on the entire surface of the glass substrate 21, an electron beam is illuminated in an electron beam exposure system so that amino groups are selectively eliminated from the glass substrate to form a pattern of amino groups arrayed on the glass substrate 21. In this example, the entire surface of the glass substrate is scanned by the electron beam so that amino-eliminated regions are formed in the form of 5-µm wide lines at intervals of 5 µm on the glass substrate 21, in other words, a 5-µm line-and-space pattern is formed owing to the presence and absence of amino groups. Incidentally, "ERIONICS" (trade mark; manufactured by JEOL Ltd.) is used as the electron beam exposure system. Further, the exposure of the electron beam is controlled at 100 µC/cm².

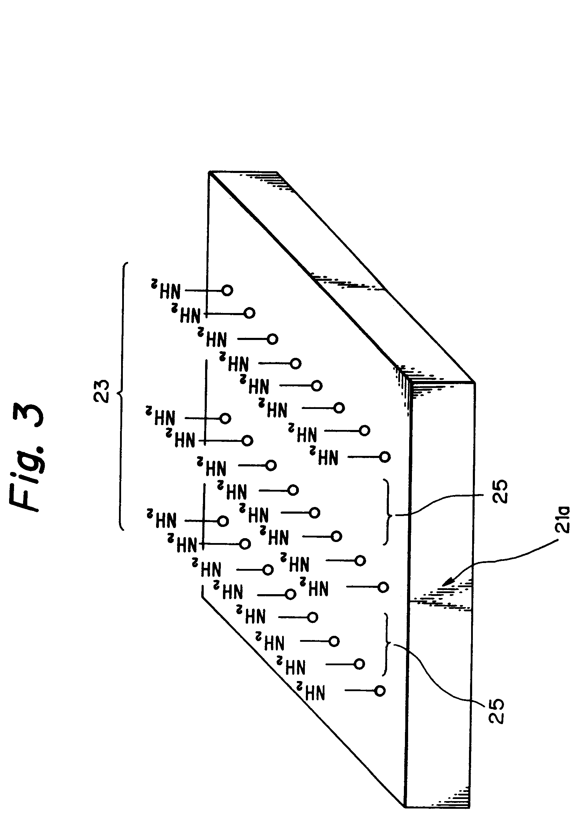

FIG. 3 schematically illustrates an array pattern of amino groups formed by illuminating the electron beam as described above. In FIG. 3, there is shown a glass plate 21a which has an array pattern 23 of amino groups and amino-eliminated regions 25.

The glass substrate 21 with the array pattern 23 of the amino groups is immersed in 10 mℓ of bicarbonate buffer of pH 8.5 placed in a sample bottle. After 3 mg of "NHS-LC-BIOTIN" are added to the sample bottle, the sample bottle is immediately shaken for 1 hour. As molecules of the biotin derivative ("NHS-LC-BIOTIN") coupled to the amino groups bound on the glass substrate 21a (see FIG. 3) during this shaking, an array pattern of molecules of the biotin derivative is formed on the substrate.

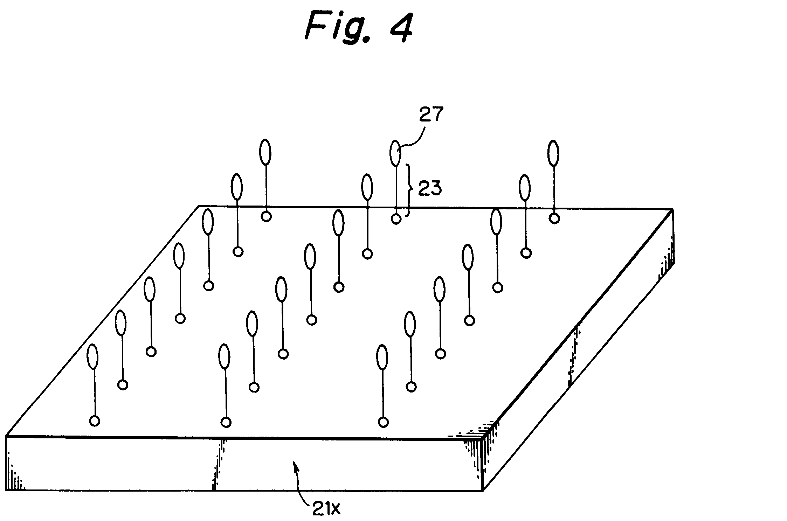

FIG. 4 schematically shows an array pattern of biotin molecules, which has been formed on a glass substrate. In FIG. 4, symbol 21x indicates the glass substrate with the biotin molecules arrayed in the pattern thereon and numeral 27 designates molecules of the biotin derivative ("NHS-LC-BIOTIN"). The biotin derivative molecules 27 are bound to the glass substrate via the array pattern 23 of the amino groups.

### A-a-3. Immobilization of proteoliposome particles on the substrate:

Next, the glass substrate 21x having the array pattern of the biotin molecules (see FIG. 4) is washed with phosphate buffer whose pH is 7.3. The glass substrate 21x is then immersed at room temperature for 2 hours in an avidin-containing bicarbonate buffer solution in which avidin D extracted from egg white (product of Funakoshi, Ltd.) has been added as avidin at the rate of 0.1 mg per mℓ of bicarbonate buffer (pH 8.5). Since avidin molecules are specifically bound to the biotin molecules on the glass substrate having the array pattern of the biotin molecules in the course of the above treatment, an array pattern of avidin molecules is formed on the glass substrate via the biotin molecules.

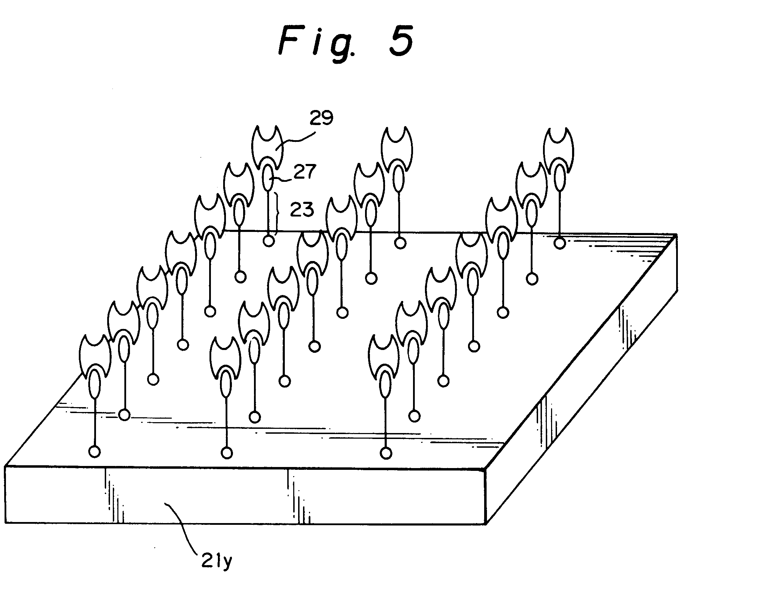

FIG. 5 schematically illustrates the array pattern of the avidin molecules, which has been formed on the glass substrate. In FIG. 5, designated at symbol 21y is the glass substrate with the array pattern of the avidine molecules bound thereon, and indicated at numeral 29 are the avidine molecules. The avidin molecules 29 are bound to the glass substrate via the biotin derivative molecules ("NHS-LC-BIOTIN") 27 and the array pattern 23 of the amino groups.

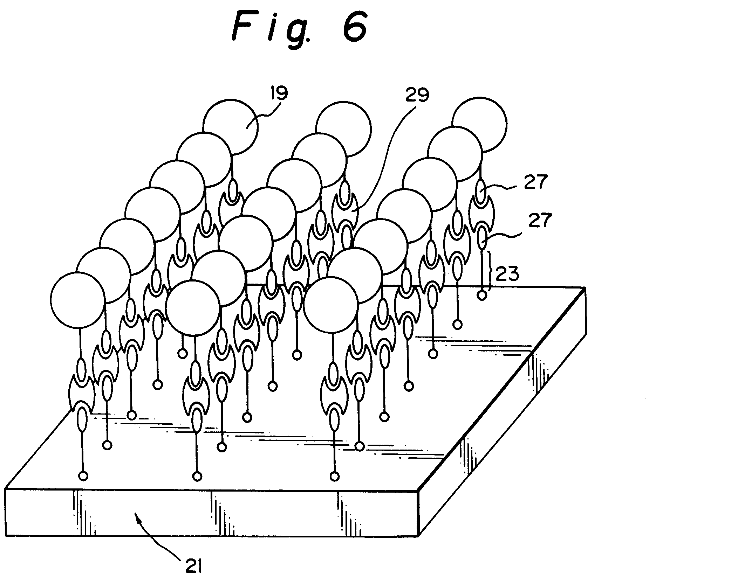

The glass substrate 21y with the array pattern of the avidin molecules bound thereon is next immersed at room temperature for 2 hours in the biotinylated proteoliposome suspension for experiments (the suspension prepared in Procedure A-a-1 described above). In the course of this treatment, the biotinylated proteoliposome particles in the biotinylated proteoliposome suspension for experiments (see FIG. 1) are immobilized via the avidin molecules on the glass substrate 21y on which the array pattern of the avidin molecules is carried. As a result, a biochip is obtained as shown in FIG. 6.

### A-a-4. Determination of the quality of the formed pattern:

To determine by a scanning electron microscope (SEM) the quality of the array pattern of proteoliposome particles on the biochip fabricated as described above (the biochip shown in FIG. 6; may hereinafter be called the "proteoliposome-immobilized substrate"), the proteoliposome-immobilized substrate is processed in accordance with the following procedures so that a sample for SEM observation is provided.

The above proteoliposome-immobilized substrate is first immersed for 24 hours in 1 vol.% glutaraldehydephosphate buffer (pH 7.3) which contains Malachite Green at the concentration of 1 wt.% and has been controlled to 4°C.

The substrate is next pulled out of the buffer, and is then immersed for 8 hours in phosphate buffer (pH 7.3) which contains OsO₄ (osmic acid) at the concentration of 1 vol.% and has been controlled to 4°C.

After the substrate is pulled out of the buffer, it is immersed in 50%, 60%, 70%, 80%, 90% and 95% aqueous methanol solutions successively for 20 minutes in each of the solutions, followed by the further immersion in methanol for 20 minutes. The substrate is hence gradually dried. Using liquefied carbon dioxide, the substrate is dried in a critical point drier.

Gold (Au) is then deposited on the substrate by sputtering, whereby a sample is provided for SEM observation. The sputtering is conducted at 1.2 KV and 5 mA for 8 minutes.

The sample for SEM observation, which has been formed as described above, is observed by SEM. The observation can confirm the formation of proteoliposome-deposited layers as lines of 2-4 µm in width extending side by side at intervals of about 5 µm on the substrate. It can also be found that the density of proteoliposome particles in the proteoliposome-deposited layers is higher than the density of liposome particles immobilized on a substrate by an antigen-antibody reaction (conventional technique). Reasons for the higher density, however, have not been ascertained.

### A-b. Example in which streptavidin and liposome are used:

An experiment - in which streptavidin is used instead of avidin D and liposome is immobilized on a base by the specific binding reaction between streptavidin and biotin - is conducted as will be described below. Streptavidin is extracted from bacteria and is different from avidin D extracted from egg white.

### A-b-1. Preparation of a suspension of biotinylated proteoliposome:

Similarly to Procedures A-a-1 described above, 5 mℓ of a chloroform solution containing DPPC at the concentration of 20 mg/mℓ and 1 mℓ of an ethanol solution containing alamethicin at the concentration of 1 mg/mℓ are placed and mixed in an eggplant type flask of 50 mℓ capacity. The solvents are then driven off under reduced pressure from the liquid mixture by a rotary evaporator.

Added next to the flask in this example is a solution of 5 mg of "4-di-10-ASP" (trade name; product of COSMO BIO CO., LTD.) as a fluorescence dye in 10 mg of phosphate buffer (pH 8.5). The contents of the flask are then stirred at 37°C by a vortex mixer so that a proteoliposome suspension is obtained. The existence of proteoliposome in the suspension is confirmed using a fluorescence microscope. Incidentally, the fluorescence dye is added because it is intended to observe the quality of the array of liposome particles by the fluorescence microscope and a high-sensitivity camera subsequent to arraying them on the substrate (this will be described in detail subsequently).

As in Procedures A-a-1 described above, proteoliposome particles are next classified to collect proteoliposome particles in a desired particle size range.

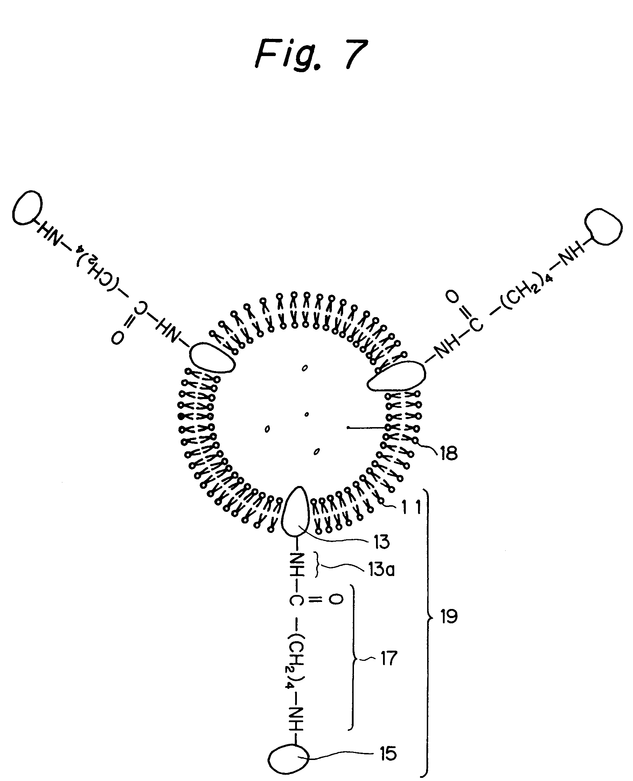

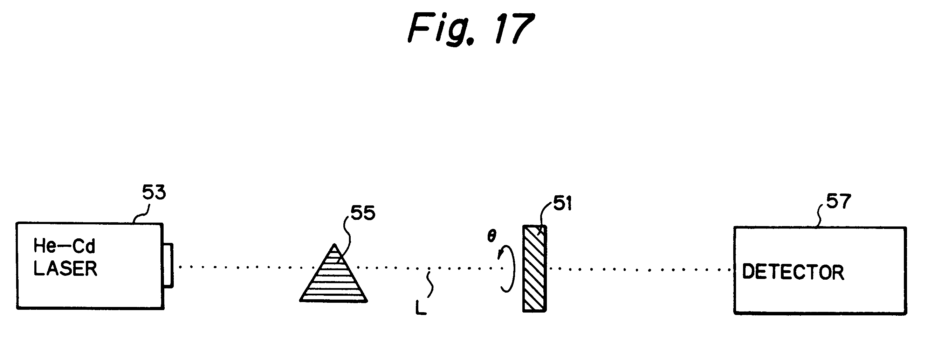

A 1-mℓ portion is next taken from a solution which has been prepared by mixing a solution of 10 mg of N-hydroxysuccinimidyl 6-(biotinamido)hexanoate ("Biotin Long Arm-NHS", trade name; product of Vector Laboratory Inc.) as biotin in 100 mℓ of dimethylformamide with 10 mℓ of bicarbonate buffer (pH 8.5), and is added to the suspension of the classified proteoliposome particles. The resultant proteoliposome suspension was stirred for 2 hours. FIG. 7 schematically shows the structure of each proteoliposome particle after the reaction. In FIG. 17, numeral 18 indicates the fluorescence dye.

The biotinylated proteoliposome suspension so obtained is next diluted tenfold in bicarbonate buffer (pH 8.5). This is provided as a biotinylated proteoliposome suspension for use in experiments.

### A-b-2. Treatment of the substrate:

By similar procedures to Procedures A-a-2 described above except that the substrate is replaced by a silicon substrate of 1 cm x 1 cm wide, the substrate is treated in an aqueous solution of aminopropyltriethoxysilane and then subjected to the prescribed washing and drying, whereby amino groups to which "Biotin Long Arm-NHS" can be bound are immobilized on the silicon substrate.

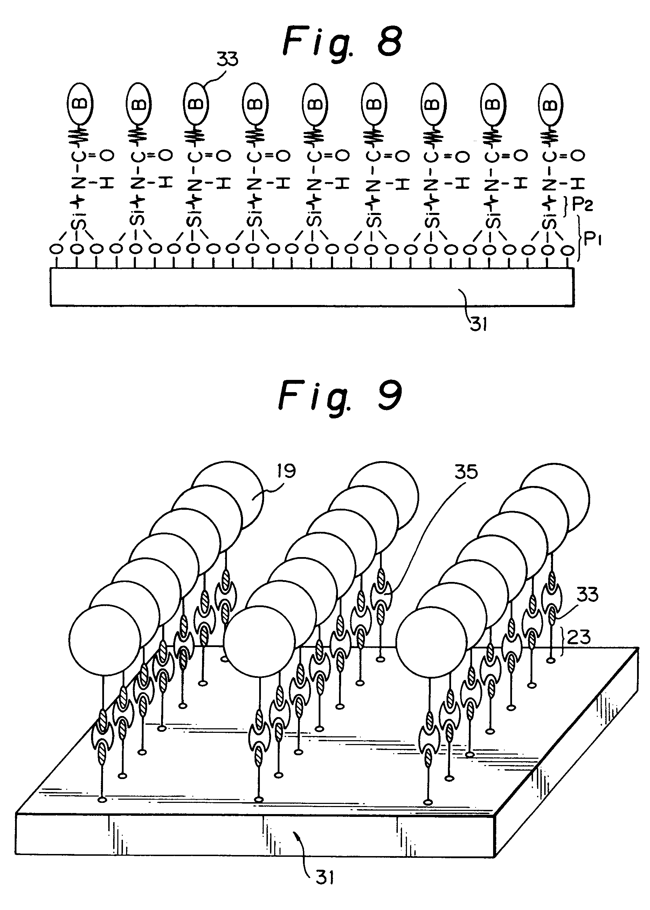

The above-described, amino-immobilized silicon substrate is next placed in a sample bottle containing a solution which has been prepared by mixing a solution of 10 mg of "Biotin Long Arm-NHS" in 100 mℓ of dimethylformamide with 10 mℓ of bicarbonate buffer (pH 8.5). They are then reacted for 2 hours while the sample bottle is ice-cooled. In the course of this reaction, molecules of the biotin derivative ("Long Arm Biotin-NHS") are bound to the entire surface of the silicon substrate via an amino group. FIG. 8 schematically illustrates this state. In FIG. 8, numerals 31 and 33 designate the silicon substrate and molecules of the biotin derivative ("Long Arm Biotin-NHS"), respectively.

From an ultraviolet exposure system ("PLA-521", trade name; manufactured by CANON INC.), light whose dominant wavelength is 290 nm is next illuminated through a photomask onto the above-described silicon substrate having biotin derivative molecules bound on the entire surface thereof. The photomask employed has a 1.2 µm line-and-space pattern. In this example, ultraviolet rays are illuminated at an output intensity of 1.3 mW for 30 minutes. By this treatment, many biotin-activated regions are formed in the form of 1.2-µm wide lines at intervals of 1.2 µm on the silicon substrate (i.e., an array pattern of biotin molecules is formed). The state of the sample at this time is as shown in PIG. 4. However, the biotin derivative is not "NHS-LC-BIOTIN" but is "Long Arm Biotin-NHS". Similar results are also obtained when ultraviolet rays, for example, with 250 nm or 260 nm dominant wavelength are used (this applies to subsequent examples where ultraviolet rays are employed).

### A-b-3. Immobilization of proteoliposome on the substrate:

The silicon substrate with the array pattern of biotin molecules is next washed with phosphate buffer whose pH is 7.5. The silicon substrate is then immersed at room temperature for 20 minutes in a phosphate buffer (pH 7.3) solution of streptavidin (product of Funakoshi, Ltd.), said solution containing streptavidin at the concentration of 0.1 mg/mℓ, Because streptavidin is bound to biotin by this treatment, a pattern of molecules of streptavidin is formed on the silicon substrate. The state of the sample at this time is as in FIG. 5. In this example, the biotin derivative in FIG. 5 is not "NHS-LC-BIOTIN" but is "Long Arm Biotin-NHS", and avidin should be replaced by streptavidin.

The silicon substrate on which the array pattern of streptavidin molecules has been formed is then immersed at room temperature for 1 hour in the biotinylated proteoliposome suspension for experiments (the suspension prepared above under A-b-1). By this treatment, biotinylated liposome particles in the biotinylated proteoliposome suspension for experiments are immobilized on the silicon substrate - which has the array pattern of streptavidin molecules - via the streptavidin molecule, respectively. FIG. 9 schematically depicts the state of the proteoliposome particles arrayed and immobilized on the silicon substrate owing to the specific binding between streptavidin and biotin. Designated at numeral 35 in FIG. 9 are molecules of streptavidin.

### A-b-4. Determination of the quality of the formed pattern:

The quality of the array pattern of the proteoliposome particles on the proteoliposome-immobilized silicon substrate fabricated as described above is next observed using a fluorescence microscope ("OPTIPHOTO 2", trade name; manufactured by NIKON CORP.) and a high-sensitivity camera ("DAS-512-G1", trade name; manufactured by Imagista Co., Ltd.). By the observation, the formation of a pattern with 1.2-µm wide, line-shaped fluorescent images arranged side by side at intervals of 1.2 µm can be confirmed.

In each of the above examples, alamethicin was used as the membrane protein to be incorporated in the lipid bilayer of liposome. The membrane protein, however, is not limited to it. various membrane proteins can be used insofar as they penetrate through the lipid bilayer and amino groups are exposed outside the liposome. For example, glycophorin has such properties [see, for example, "Saibo no Bunshi Seibutsugaku (Molecular Biology of Cells)", 271, Kyoikusha, Inc. (1987)] so that it can be used as a membrane protein in the present invention.

In addition, liposome which can be arrayed and immobilized on a base in this invention is not limited to those described above. Liposomes of various forms, for example, liposomes formed of a biosubstance, liposomes formed of both a biosubstance and a biosubstance analogue, and liposomes with another suitable substance bound thereto to impart a function can all be used.

### A-c. Example in which avidin and a protein was used:

Each of the two examples described above illustrates the provision of a biochip by arraying and immobilizing liposome particles on a substrate. The following example illustrates the provision of a biochip by arraying and immobilizing protein molecules as a substitute for liposome particles on a substrate in accordance the specific binding reaction between avidin and biotin.

### A-c-1. Treatment of the substrate:

A silicon substrate of 1 cm x 1 cm wide is immersed at room temperature for 1 hour in a 1:4 (by volume) mixed solution of 31 wt.% hydrogen peroxide and 97 wt.% sulfuric acid. By procedures similar to Procedures A-a-2, the silicon substrate is next treated in an aqueous solution of aminopropyltriethoxysilane, followed by the prescribed washing and drying. As a result, amino groups to which "Biotin Long Arm-NHS" can be bound are immobilized on the silicon substrate. Incidentally, the treatment with the mixed solution of hydrogen peroxide and sulfuric acid is conducted to positively apply OH groups which can facilitate the binding of a silane coupling agent (aminopropyltriethoxysilane in this example) on the surface of the silicon substrate. Of course, this treatment with the mixed solution is not essential.

In this example, the above-described amino-immobilized silicon substrate is placed, as in Procedures A-b-2, in a sample bottle which contains a mixed solution consisting of a solution of 10 mg of "Biotin Long Arm-NHS" in 100 mℓ of dimethylformamide and 10 mℓ of bicarbonate buffer (pH 8.5). While the sample bottle is ice-cooled, they are reacted for 2 hours. By the reaction, molecules of the biotin derivative ("Long Arm Biotin-NHS") are bound on the entire surface of the silicon substrate via an amino group. The state of the sample at this time is as shown in FIG. 8.

From an ultraviolet exposure system ("PLA-521", trade name; manufactured by CANON INC.), light whose dominant wavelength is 290 nm is next illuminated through a photomask onto the above-described silicon substrate having biotin derivative molecules bound on the entire surface thereof. The photomask employed has a 1.2 µm line-and-space pattern. Ultraviolet rays are illuminated at an output intensity of 1.3 mW for 30 minutes. By this treatment, many biotin-activated regions are formed in the form of 1.2-µm wide lines at intervals of 1.2 µm on the silicon substrate.

### A-c-2. Immobilization of a protein on the substrate:

As the protein to be arrayed and immobilized on the substrate, β-phycoerythrin, a fluorescence protein, is employed in this example. To conduct the immobilization of β-phycoerythrin on the substrate by using the specific binding reaction between avidin and biotin, β-phycoerythrin has to be bound to avidin first of all. Commercial β-phycoerythrin-bound avidin (product of Molecular Probe Inc.) is used in this example. Incidentally, the binding of β-phycoerythrin to avidin can be effected, for example, by the process disclosed in J. Cell Biol. **93**, 981-986 (1982). According to this process, various proteins, without the need to be limited to β-phycoerythrin, can be bound to avidin as long as they have amino groups (this also applies to streptavidin to be described subsequently).

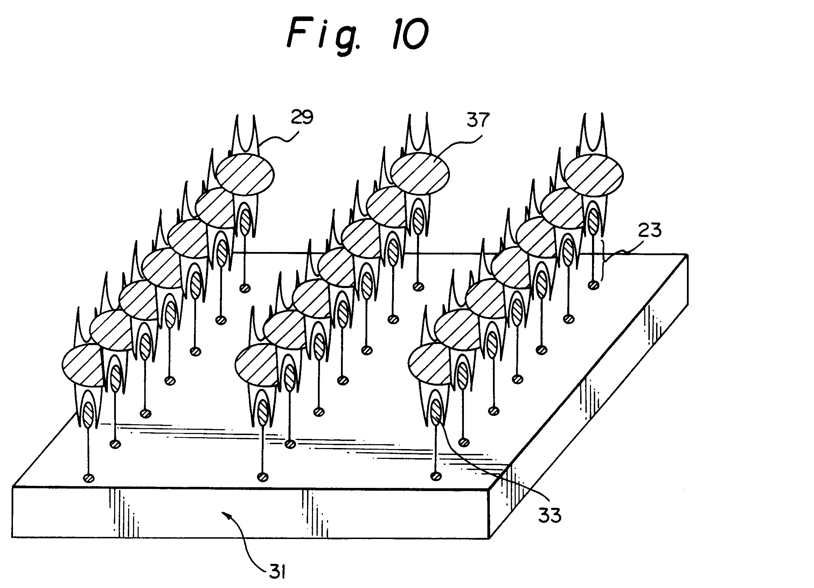

The above-described silicon substrate on which the array pattern of biotin molecules has been formed is immersed at room temperature for 20 minutes in a solution of 1 mg of β-phycoerythrin-bound avidin in 10 mℓ of phosphate buffer (pH 7.2). As β-phycoerythrin molecules are coupled to the array pattern of biotin molecules via an avidin molecule by the above treatment, the β-phycoerythrin molecules are arrayed and immobilized on the silicon substrate. FIG. 10 schematically illustrates the state of the arrayed immobilization of β-phycoerythrin molecules 37 on the silicon substrate by the specific binding between avidin and biotin.

### A-c-3. Determination of the quality of the formed pattern:

The quality of the array pattern of the β-phycoerythrin molecules on the β-phycoerythrin-immobilized silicon substrate fabricated as described above is next observed using a fluorescence microscope ("OPTIPHOTO 2", trade name; manufactured by NIKON CORP.) and a high-sensitivity camera ("DAS-512-G1", trade name; manufactured by Imagista Co., Ltd.). By the observation, the formation of a pattern with 1.2-µm wide, line-shaped fluorescent images arranged side by side at intervals of 1.2 µm can be confirmed.

### A-d. Example in which streptavidin and a protein were used:

In Procedures A-c described above, the arrayed immobilization of the protein on the base was conducted using the specific binding reaction between avidin and biotin. In this example, a protein is arrayed and immobilized on a silicon substrate by using the specific binding reaction between streptavidin and biotin.

An experiment and the determination of the quality of a formed pattern, therefore, can be conducted by the same procedures as Procedures A-c except for the use of β-phycoerythrin-bound streptavidin (product of Molecular Probe Inc.) instead of the β-phycoerythrin-bound avidin (product of Molecular Probe Inc.) employed in Procedures A-c. As a result, it has been found that the use of streptavidin also enables to array and immobilize a protein as in the use of avidin.

In each of the examples described above under A-c and A-d, β-phycoerythrin was used as a protein to be arrayed on a base. The protein which can be arrayed and immobilized on a base by the process according each aspect of this invention, however, is not limited to the above protein. A variety of proteins can be used, with those containing an amino group being particularly preferred.

### B. Example of the arraying process of ultrafine particles:

A description will next be made of the process according to the third aspect of this invention for arraying ultrafine particles. In this example, ultrafine gold particles and a silicon substrate are used as ultrafine particles and a base, respectively.

### B-1. Treatment of the substrate:

In accordance with the procedures described above under A-b-2, a silicon substrate of 1 cm x 1 cm wide is treated with the silane coupling agent (aminopropyltriethoxysilane), followed by prescribed washing and drying. Following the procedures described above under A-b-2, molecules of "Long Arm Biotin-NHS" are bound to the substrate. Further, ultraviolet rays are selectively illuminated through a photomask onto the molecules of "Long Arm Biotin-NHS". By this treatment, many biotin-activated regions are formed in the form of 1.2-µm wide lines at intervals of 1.2 µm on the silicon substrate (i.e., an array pattern of biotin molecules is formed).

### B-2. Binding of ultrafine particles with avidin:

Ultrafine particle-avidin molecule conjugates are provided in this example as will be described below. In this example, avidin D (product of Funakoshi, Ltd.) is used as avidin while ultrafine gold (Au) particles are used as ultrafine particles. The ultrafine gold particles are obtained by reducing an aqueous solution of chloroauric acid with a saturated solution of white phosphorus solution in diethyl ether and also with ascorbic acid or citric acid in accordance with the procedures reported by J. Demey et al. in Proc. Natl. Acad. Sci. USA, **79**, 1898 (1982). The ultrafine gold particles and avidin D are placed in an aqueous solution of a mixture consisting of 50 mM of Tris-HCl (pH 8.5) and 50 mM of NaCl, and are reacted for 24 hours to bind ultrafine gold particles on molecules of avidin D. The ultrafine gold particle-avidin conjugates will hereinafter be called "colloidal gold-avidin particles" for the sake of convenience of description.

### B-3. Coupling of colloidal gold-avidin particles with biotin molecules on the substrate:

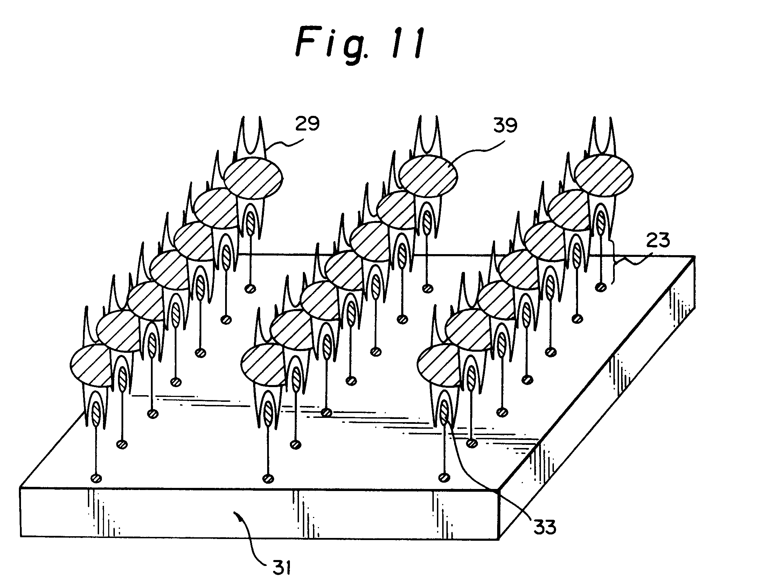

A 2 µl portion of the colloidal gold-avidin solution prepared as described above is next diluted to 2 mℓ with phosphate buffer (pH 7.4). The above-described sample substrate (the substrate with biotin molecules arrayed in the pattern) is immersed at room temperature for 1 hour in the diluted colloidal gold-avidin solution prepared as described above. As a result, as is illustrated in FIG. 11, ultrafine particles (colloidal metal particles) 39 bound to avidin molecules 29 are bound to "Long Arm Biotin-NHS" molecules 33 on a silicon substrate 31 via an avidin molecule, respectively, whereby the ultrafine gold particles are successfully arrayed on the substrate.

### B-4. Confirmation of the formation of an array pattern of ultrafine particles:

By SCM observation of the ultrafine-particle-arrayed substrate obtained as described above, the formation of a 1.2 µm line-and-space pattern with ultrafine metal particles on the substrate can be confirmed.

In the above-described example of the arraying process of ultrafine particles, the ultrafine gold particles were arrayed and immobilized on the substrate by using the specific binding reaction between avidin and biotin. Effects similar to those obtained in the above example can also be obtained when the specific binding reaction between streptavidin and biotin is used.

### C. Example of the process for the formation of fine conductive paths:

The process according to the fourth aspect of this invention for the formation of ultrafine conductive paths will next be described. This aspect will be described on the basis of an example in which a silicon substrate is used as a base and conductive gold (Au) paths are formed on the silicon substrate.

### C-1. Treatment of the substrate:

A quartz substrate of 1 cm x 1 cm wide is refluxed for 4 hours in ethanol which contains aminopropyltriethoxysilane ("LS-3150", trade name; product of Shin-Etsu Silicone Co., Ltd.) at the concentration of 10% by volume. After the quartz substrate is pulled out of the above-described solution, the substrate is washed with ethanol and then with hot water. Owing to this series of treatments, amino groups to which biotin molecules can be bound are immobilized on the surface of the quartz substrate.

The above-described, amino-immobilized quartz substrate is next placed in a sample bottle which contains 10 mℓ of bicarbonate buffer (pH 8.5). To the sample bottle, 100 µl of a 30 mg/mℓ solution of "NHS-LONG ARM-BIOTIN" in dimethylformamide (DMF) are added. The sample bottle is then immediately shaken for about 1 hour so that the amino groups on the quartz substrate are reacted with molecules of "NHS-LONG ARM-BIOTIN". After the treatment, the quartz substrate is pulled out of the sample bottle, followed by washing with purified water. By such a series of treatments, the molecules of "NHS-LONG ARM-BIOTIN" are bound to the entire surface of the quartz substrate via an amino group. Incidentally, "NHS-LONG ARM-BIOTIN" is a trade name for a biotin derivative produced by Funakoshi, Ltd.

Next, an electron beam is selectively illuminated onto the above-described quartz substrate with biotin molecules bound on the entire surface of the substrate. In this example, the quartz substrate is scanned by the electron beam so that a number of line-shaped, electron-beam illuminated regions and electron-beam non-illuminated regions, both of 25 nm in width, are alternately formed. As an electron beam exposure system, "ERIONICS" (trade mark; manufactured by JEOL Ltd.) is used. The exposure of the electron beam is controlled at 100 µC/cm².

In this example, amino groups are immobilized on the entire surface of the quartz substrate, biotin molecules are immobilized on the amino groups and, then, biotin molecules and amino groups are selectively eliminated by the electron beam. It is, however, possible to selectively eliminate amino groups by an electron beam immediately after the immobilization of amino groups on the entire surface of the quartz substrate and, then, to place the substrate in the sample bottle to couple biotin molecules.

### C-2. Binding of streptavidin, which has a colloidal metal, with biotin:

As streptavidin having a colloidal metal, a colloidal gold (Au)-avidin conjugate is used in this example. Described specifically, gold colloid-streptavidin called "streptavidin-gold conjugate, EM 15 nm" ("RL-1121-50", trade name; product of Biocell Laboratories, Inc.) is used.

A 2 µl portion of the colloidal gold-streptavidin solution is taken and, then, is diluted to 2 mℓ with phosphate buffer (pH 7.4).

In the diluted colloidal gold-streptavidin solution prepared as described above, the quartz substrate on which the array pattern of biotin molecules has been formed is immersed at room temperature for 1 hour. As a result, the colloidal gold particles bound to the streptavidin molecules, respectively, are bound via a streptavidin molecule to the array pattern of streptavidin formed on the quartz substrate, whereby conductive paths composed of an array pattern of colloidal metal particles consistent with the array pattern of biotin molecules are formed on the quartz substrate.

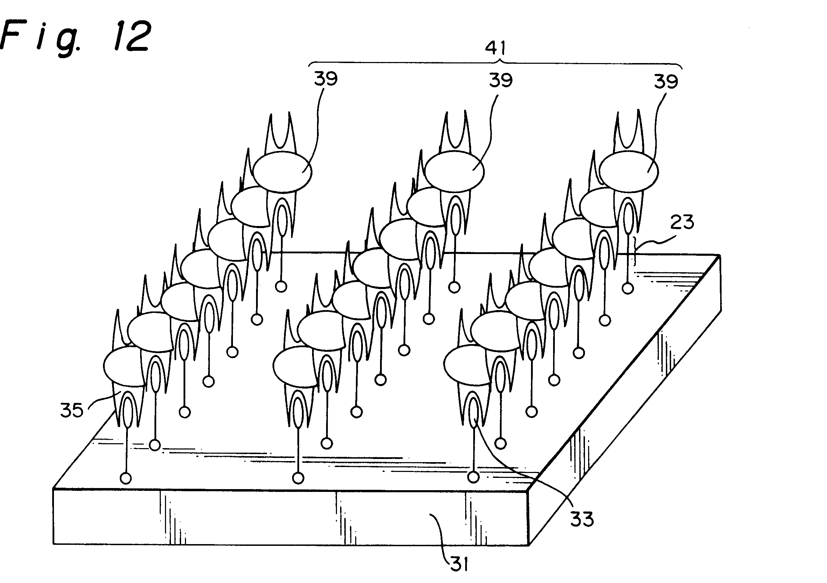

FIG. 12 is a perspective view in which an essential part of conductive metal paths 41 composed of an array pattern of colloidal gold particles 39 formed as described above is schematically shown on an enlarged scale.

### C-3. Characteristics of the thus-formed conductive paths:

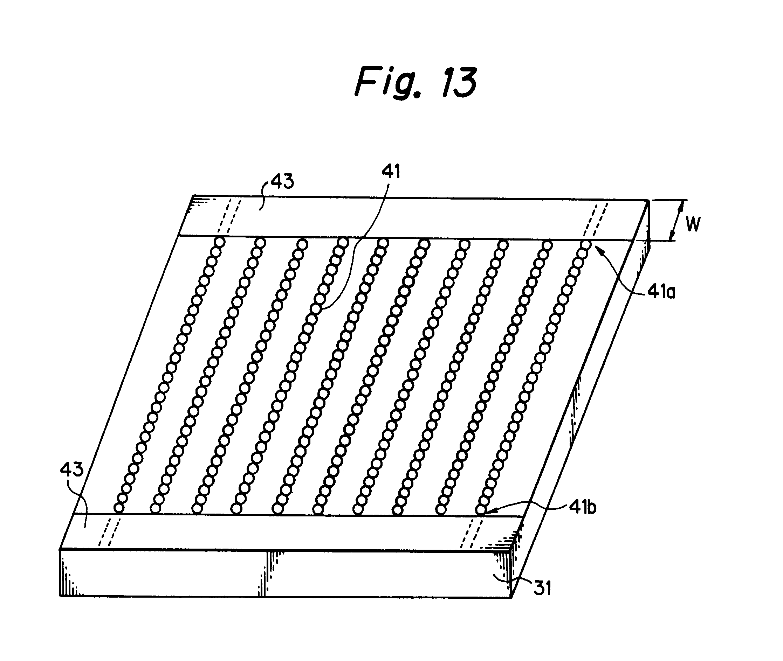

As is shown in FIG. 13, electrodes 43 (I-V characteristics measuring electrodes 43) connected to both longitudinal ends 41a,41b of conductive paths 41 are next formed. The lengthwise direction of each electrode 43 is perpendicular to the longitudinal directions of the respective conductive paths 41. Its width W is 2 mm. The I-V characteristics of the conductive paths 41 are then measured. Incidentally, the I-V characteristics measuring electrodes 43 are formed of a vacuum-evaporated gold film in this example.

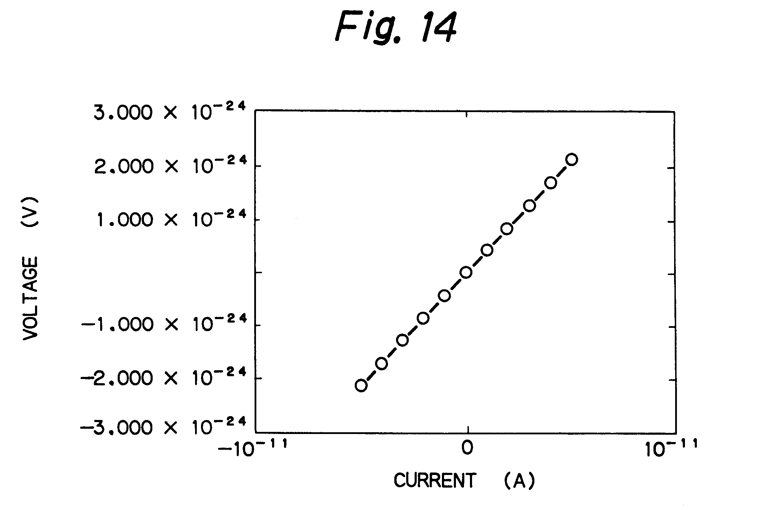

FIG. 14 is a characteristic diagram in which the results of the measurement, namely, voltages (V) are plotted along the axis of ordinates and currents I (A) is plotted along the axis of abscissas. As is evident from FIG. 14, it is understood that the conductive paths 41 has good ohmic characteristics. From this, it is understood that formation of conductive paths on the nanometer order is feasible by the process according to the fourth aspect of this invention for the formation of ultrafine conductive paths.

In the above-described example of the process for the formation of ultrafine conductive paths, conductive paths were formed by arraying and immobilizing colloidal gold particles on a silicon substrate while making use of the specific binding reaction between streptavidin and biotin. Advantageous effects similar to those obtained in the above example can also be obtained when the specific binding reaction between avidin and biotin are used. Further, formation of conductive paths other than gold paths can also be expected from the use of other colloidal metal particles.

### D. Example of the process for the fabrication of a polarizer:

A description will next be made of one example of the process for the fabrication of a polarizer.

### D-1. Treatment of a substrate for the fabrication of a polarizer:

A quartz substrate of 4 cm x 4 cm wide is refluxed for 4 hours in ethanol which contains aminopropylethoxysilane ("LS-3150", trade name; product of Shin-Etsu Silicone Co., Ltd.) at the concentration of 10% by weight. After the substrate is pulled out of the solution and then washed with ethanol, the substrate is washed further with hot water. By this series of treatments, amino groups to which molecules of "NHS-LONG ARM-BIOTIN" (product of Funakoshi, Ltd.) to be described subsequently can be bound are immobilized on the quartz substrate. The state of the sample at this stage is similar to that shown in FIG. 2.

The quartz substrate whose surface has been modified as described above is next immersed in 10 mℓ of bicarbonate buffer (pH 8.5) placed in a sample bottle. Then, 100 µℓ of a biotin-containing DMS solution, said solution containing "NHS-LONG ARM-BIOTIN" at the concentration of 30 mg/mℓ, are added to the sample bottle. Immediately after the addition, the sample bottle is shaken so that they are reacted for about 1 hour. After the resulting substrate is washed with purified water, an electron beam is selectively illuminated onto the substrate by using an electron beam exposure system (manufactured by ERIONICS Company) so that a 200-nm line-and-space pattern is formed. By that operation, a pattern of narrow lines made of biotin molecules and having a width of 200 nm is formed at prescribed line-to-line intervals (200 nm intervals) on the substrate. The state of the sample at this stage is similar to that shown in FIG. 4.

### D-2. Immobilization of colloidal gold-streptavidin conjugate on a substrate:

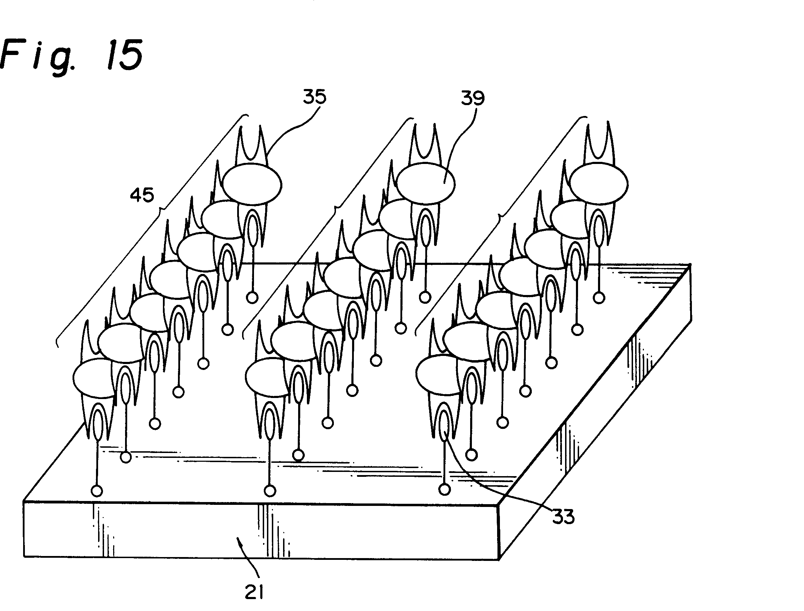

A 2 µℓ portion of the colloidal gold-streptavidin solution (Streptavidin Gold Conjugate, EM 15 nm), which was employed in the above-described example of the process for the formation of ultrafine conductive paths, is taken and then diluted to 2 mℓ with phosphate buffer (pH 7.4). The above-described substrate on which the pattern of biotin molecules has been formed is immersed at room temperature for 1 hour in the diluted colloidal gold-streptavidin solution prepared as described above. As a result, as is illustrated in FIG. 15, the colloidal gold particles 39 of the colloidal gold-streptavidin conjugates are bound via a streptavidin molecule to biotin molecules 33 formed beforehand in a pattern on the substrate so that a number of ultrafine conductive paths 45 for the formation of a polarizer are formed at prescribed intervals (200 nm in this case) on a substrate 21. As a consequence, the polarizer of this example has been formed.

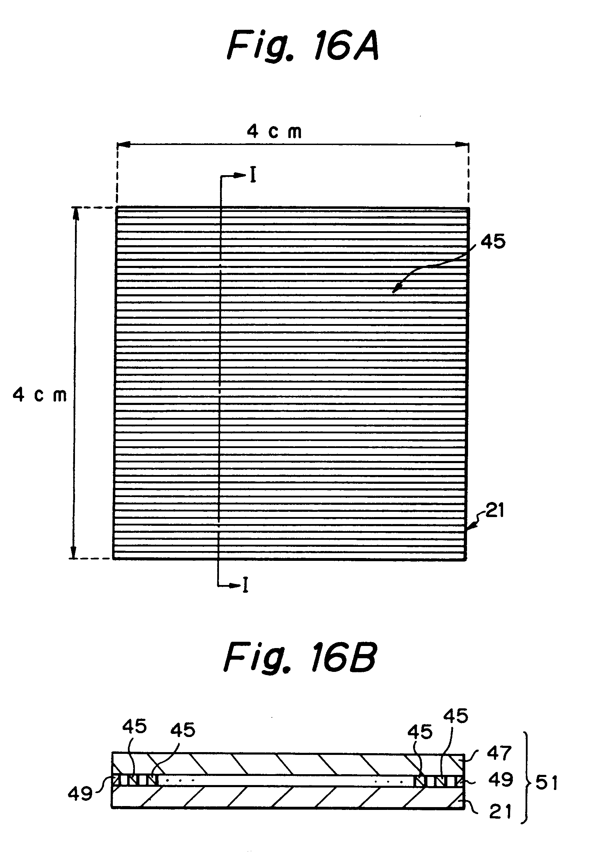

In order to protect the ultrafine conductive paths 45, the polarizer of this example which has been fabricated as described above is formed, as illustrated in FIGS. 16(A) and 16(B), into a sandwich structure in this example so that an additional quartz substrate 47 is superposed on one side of the substrate 21, said side carrying the ultrafine conductive paths 45 formed thereon, to sandwich the find conductive paths 45 therebetween. Further, the substrate 21 and the additional quartz substrate 47 are fixed together by an adhesive 49. Needless to say, the structure of the polarizer 51 is not limited to that depicted in FIG. 16.

### D-3. Determination of characteristics of the polarizer:

The polarizing characteristics of the polarizer 51, which has been fabricated by the process of the above example and is illustrated in FIG. 16, are then measured as will be described below.

### D-3-1. Measurement of characteristics by using one polarizer:

Characteristics when one polarizer 51 fabricated by the process of the above example is used are measured first. For this purpose, as is illustrated in FIG. 17, the polarizer 51 is arranged between a polarizing prism 55 and a detector 57 in a measurement system in which a He-Cd laser 53 of 325 nm wavelength as a light source, the polarizing prism 55 and the detector 57 are disposed along a straight line. The intensity of light transmitted upon rotation of the polarizer 51 about an optical axis L as an axis of rotation is measured. In the present example, an angle of rotation upon clockwise rotation of the polarizer 51 as viewed from the side of the polarizing prism 55 will be regarded as a positive angle while an angle of rotation upon counterclockwise rotation will be regarded as a negative angle. In this example, the measurement is conducted by rotating the polarizer 51 in a range of from about -100 degrees to about +300 degrees. The term "the rotation angle of 0 degree" indicates that the direction of polarization for light outputted from the polarizing prism 55 is parallel to the ultrafine conductive paths 45 of the polarizer 51.

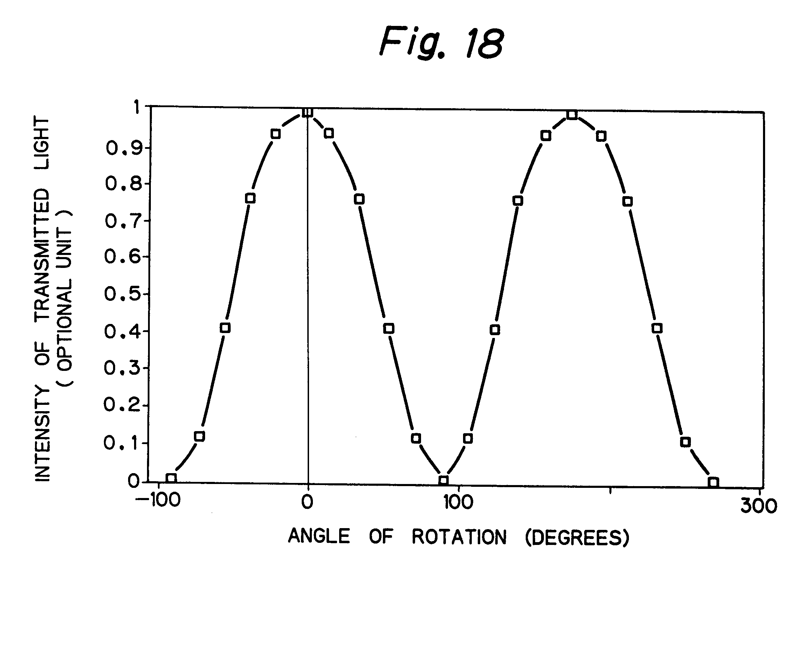

FIG. 18 diagrammatically illustrates the results of the measurement by plotting angles of rotation (degrees) along the axis of abscissas and the intensities of transmitted light (values normalized based on the intensity at the rotation angle of 0 degree) along the axis of ordinates.

As is apparent from FIG. 18, it is understood that the polarizer 51 fabricated by the process according to the present invention shows good polarizing characteristics for light in the ultraviolet range (light of 325 nm wavelength in this example).

### D-3-2. Where two polarizers are used:

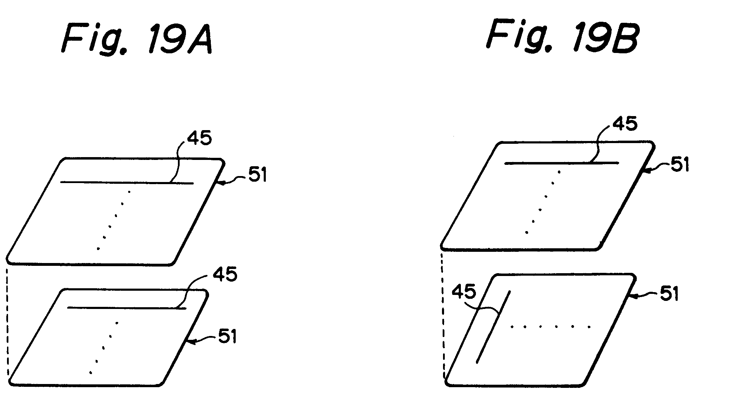

Measured next will be light transmission characteristics when two polarizers fabricated by the process of the above example are superposed in an open state [see FIG. 19(A)] and also when the two polarizers are superposed in a closed state [see FIG. 19(B)]. The measurement is conducted by arranging the polarizers in an open or closed state at the position where the polarizer 51 of the measurement system shown in FIG. 17 is supposed to be placed.

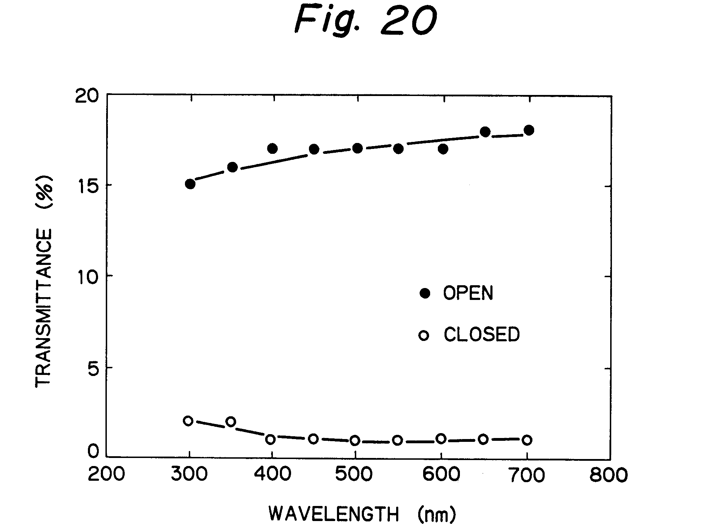

The results of the measurement are shown in FIG. 20. As is clear from FIG. 20, it is understood that the polarizers fabricated by the process of the above example exhibit good polarizing characteristics.

In the above-described process for the fabrication of a polarizer, it is clear that the width of the ultrafine conductive paths 45 and the intervals between the ultrafine conductive paths 45 can be modified depending on the wavelength of light to be polarized by the polarizer. In the above example, selective illumination of an electron beam was employed as a method for forming an array pattern of biotin molecules on a substrate. This is to facilitate the setting of the intervals between ultrafine conductive paths at such a very small dimension that permits polarization of light in the ultraviolet range. This is also feasible by using a laser beam. Other light source such as an ultraviolet ray source, however, can be used where it is unnecessary to make the intervals of ultrafine conductive paths so narrow.

The present invention has been described above on the basis of the examples of the respective aspects. It should however be borne in mind that the present invention is not limited to the above examples.

In each of the examples described above, an array pattern of biotin molecules was formed using an electron beam or ultraviolet rays. In some instances, the formation of such an array pattern, however, can be conducted by selective illumination of a laser beam or by illumination of X-rays through an X-ray mask having a prescribed pattern therein.

Biotin derivatives usable in the present invention are not limited to those described above. Other illustrative usable biotin derivatives include N-hydroxysuccinimide imminobiotin (for example, "NHS-Iminobiotin", trade name; product of Pierce Chemical Company), sulfosuccinimidyl 2-(biotinamido)ethyl-1,3-dithiopropionate (for example, "NHS-SS-Biotin", trade name; product of Pierce Chemical Company), and sulfosuccinimidobiotin (for example, "Sulfo-NHS-Biotin", trade name; product of Pierce Chemical Company).

In each of the examples described above, a base (glass substrate, silicon substrate, quartz substrate or the like) was pre-treated with a silane coupling agent having an amino group. A biotin derivative, however, may be bound to a base via an OH group or the like which has been bound beforehand to the surface of the base. In this case, it is also possible to more firmly immobilize ultrasmall objects on the base than the arrayed immobilization of the ultrasmall objects on the base in accordance with the antigen-antibody reaction. This pre-treatment, therefore, can be omitted depending on the design. Nevertheless, it is suitable to apply this pre-treatment because ultrasmall objects can be immobilized firmly and at a higher density on a base. The manner of such pre-treatment is not limited to the manners employed in the examples but can be modified to a suitable manner depending on the kind of the base.

According to the first to fifth aspects of the present invention, immobilization of ultrasmall objects in a prescribed array pattern on a base is conducted by making use of the specific binding reaction between avidin and biotin and/or the specific binding reaction between streptavidin and biotin. The immobilization of the ultrasmall objects on the base, therefore, can be effected more firmly compared with immobilization of the ultrasmall objects on the base in accordance with an antigen-antibody reaction. Moreover, as is evident from the results of the above-described experiments, the ultrasmall objects can be immobilized at a higher density than the immobilization of the ultrasmall objects on the base by the antibody-antigen reaction.

Where biotin molecules are bound to a base via an amino group, each biotin molecule and the base are bound by a covalent bond so that the strength of the binding other than the coupling between each biotin molecule and its corresponding avidin molecule can be ensured. Accordingly, the specific binding between avidin and biotin and/or the specific binding between streptavidin and biotin can be used more effectively.

Where an electron beam or the like is selectively illuminated onto amino groups or biotin molecules, binding activity remains at the amino groups or biotin molecules in regions not exposed to the electron beam or the like. The regions where binding activity remains, therefore, function as a pattern of biotin molecules. As a consequence, the biotin molecules can be patterned like a resist widely employed in lithography. Moreover, the resolution of the array pattern of biotin molecules can be directly controlled, for example, depending on the diameter of the electron beam so that much finer patterns can be provided compared with conventional resist patterns. Since ultrasmall objects are arrayed in accordance with such a fine pattern of biotin molecules, it is possible to form an array pattern which has a line width as small as several tens nanometers and has not been available by the conventional lithography.

According to the process of the second aspect of this invention for the fabrication of a biochip, various liposome capable of responding to various external information - such as light, taste or smell - or proteins having various functions can be immobilized on a substrate more firmly and at a higher density than conventional processes. Construction of multi-function biochips simulating processing mechanisms of information by organisms can be expected. This process is also expected to contribute to the construction of biocomputers and also to their input and output devices.

In the process of the second aspect of this invention for the fabrication of a biochip, biotin molecules and liposome particles can be coupled firmly where a membrane protein molecule is incorporated in each liposome particle and a biotin molecule is bound to the liposome particle via the membrane protein molecule. Accordingly, the specific binding between biotin and avidin or the specific binding between biotin and streptavidin can be used effectively.

According to the process of the third aspect of this invention for arraying ultrafine particles, the ultrafine particles can be arrayed and immobilized firmly and, moreover, easily on a base.

According to the process of the fourth aspect of this invention for the formation of ultrafine conductive paths, it is possible to form ultrafine conductive paths having a line width not greater than several tens nanometers.

Further, the process of the fifth aspect of this invention for the fabrication of a polarizer makes it possible to obtain a polarizer in which the intervals between ultrafine conductive paths are narrower than those in conventional polarizers. It is therefore possible to fabricate a polarizer which has many ultrafine conductive paths arranged side by side thereon and permits polarization of light in the visible range or even in the infrared range, said polarization having been considered difficult conventionally.

## Claims

1. A process for arraying ultrasmall objects, which comprises immobilizing the ultrasmall objects in a predetermined pattern on a base by using at least one of a specific binding reaction between avidin and biotin and a specific binding reaction between streptavidin and biotin.

2. The process of claim 1, wherein the process comprises:
forming the predetermined pattern with first biotin molecules on the base;
binding second biotin molecules to the ultrasmall objects, respectively; and
coupling via a streptavidin or avidine molecule the ultrasmall objects, to which the second biotin molecules have been bound respectively, to the respective first biotin molecules formed in the predetermined pattern on the base.

3. The process of claim 2, wherein the predetermined pattern is formed by binding amino groups to an entire surface of the base, patterning the bound amino groups and then coupling the first biotin molecules to the patterned amino groups, respectively; or by binding amino groups to the entire surface of the base, coupling biotin molecules to the bound amino groups, respectively and then patterning the coupled biotin molecules.

4. The process of claim 3, wherein the predetermined pattern is formed by selectively illuminating an electron beam, a laser beam, ultraviolet rays or X-rays to the bound amino groups or coupled biotin molecules to pattern the bound amino groups or coupled biotin molecules.

5. The process of claim 1, wherein the process comprises:
forming the predetermined pattern with biotin molecules on the base;
binding streptavidin or avidine molecules to the ultrasmall objects, respectively; and
coupling the ultrasmall objects, to which the streptavidin or avidine molecules have been bound respectively, to the respective biotin molecules formed in the predetermined pattern on the base.

6. The process of claim 5, wherein the predetermined pattern is formed by binding amino groups to an entire surface of the base, patterning the bound amino groups and then coupling the first biotin molecules to the patterned amino groups, respectively; or by binding amino groups to the entire surface of the base, coupling biotin molecules to the bound amino groups, respectively and then patterning the coupled biotin molecules.

7. The process of claim 6, wherein the predetermined pattern is formed by selectively illuminating an electron beam, a laser beam, ultraviolet rays or X-rays to the bound amino groups or coupled biotin molecules to pattern the bound amino groups or coupled biotin molecules.

8. A process for fabricating a biochip, which comprises immobilizing protein molecules in a predetermined pattern on a base by using at least one of a specific binding reaction between avidin and biotin and a specific binding reaction between streptavidin and biotin.

9. The process of claim 8, wherein the protein molecules are liposome particles, which are formed of at least one of biosubstances and biosubstance analogues.

10. The process of claim 9, wherein at least one membrane protein molecule is incorporated as a biotin-binding site in each of the liposome particles and a biotin molecule is coupled to the membrane protein molecule on the side of the liposome particle.

11. A process for arraying ultrafine particles, which comprises immobilizing the ultrafine particles in a predetermined pattern on a base by using at least one of a specific binding reaction between avidin and biotin and a specific binding reaction between streptavidin and biotin.

12. A process for forming ultrafine metal paths, which comprises immobilizing colloidal metal particles in a predetermined pattern on a base by using at least one of a specific binding reaction between avidin and biotin and a specific binding reaction between streptavidin and biotin.

13. A process for fabricating a polarizer, which comprises:
forming a pattern of narrow lines, which are arranged at predetermined intervals, with first biotin molecules on a base;
binding second biotin molecules to colloidal metal particles, respectively; and
coupling via a streptavidin or avidine molecule the colloidal metal particles, to which the second biotin molecules have been bound respectively, to the respective first biotin molecules formed in the pattern of the narrow lines on the base.

14. A process for fabricating a polarizer, which comprises:
forming a pattern of narrow lines, which are arranged at predetermined intervals, with biotin molecules on a base;
binding streptavidin or avidine molecules to colloidal metal particles, respectively; and
coupling the colloidal metal particles, to which the streptavidin or avidine molecules have been bound respectively, to the respective biotin molecules formed in the pattern of the narrow lines on the base.
